# NEUE EUROPÄISCHE PATENTSCHRIFT

(11) **EP 0 481 294 B2**
(45) Veröffentlichungstag und Bekanntmachung des Hinweises auf die Entscheidung über den Einspruch: **11.04.2001**
(45) Hinweis auf die Patenterteilung: 02.08.1995
(21) Anmeldenummer: 91116899.5
(22) Anmeldetag: 04.10.1991
(51) Int. Cl.: A61K 31/195, A61K 9/20

(54) **Feste, schnell-lösliche Arzneimittelzubereitung enthaltend N-Acetylcystein**
Solid fast-dissolving pharmaceutical preparation containing N-acetyl-cysteine
Préparation pharmaceutique solide à dissolution rapide contenant de la N-acétylcystéine

(30) Priorität: 19.10.1990 CH 334590
(43) Veröffentlichungstag der Anmeldung: 22.04.1992
(73) Patentinhaber: SPIRIG AG PHARMAZEUTISCHE PRÄPARATE, CH-4622 Egerkingen (CH)
(72) Erfinder: Juch, Rolf-Dieter, CH-4612 Wangen b. Olten (CH); Birrenbach, Gerd, Dr., CH-4616 Kappel (SO) (CH); Pflugshaupt, Christian, Dr., CH-4616 Hägendorf (CH)
(74) Vertreter: Braun, André, jr.

(56) Entgegenhaltungen:
- EP-A- 0 209 957
- EP-A- 0 281 200
- EP-A- 0 339 508
- EP-A- 0 340 662
- FR-A- 2 437 834
- FR-A- 2 452 929
- MANUFACTERING CHEMIST Bd. 58, Nr. 6, Juni 1987, WOOLWICH/LONDON Seiten 55 - 59; S. BURGESS: 'sorbitol instant -a unique excipient'
- DRUG DEVELOPMENT AND INDUSTRIAL PHARMACY, 12(11-13) [1986], Seiten 2061-2089
- IKS Monatsbericht 9/1990
- BIOPHARMACEUTICS & DRUG DISPOSITION, Vol. 12, pp. 343-353 [1991]

## Beschreibung

Die vorliegende Erfindung betrifft eine oral verabreichbare, feste und schnell-lösliche N-Acetylcystein enthaltende Arzneimittelzubereitung, die sich sowohl zum Auflösen in Wasser oder in wässriger Lösung, als auch zum Lutschen oder Schlucken eignet.

N-Acetylcystein ist als sehr verträglicher pharmakologisch aktiver Wirkstoff mit hauptsächlich schleimlösender Wirkung bekannt. Ueblicherweise werden diese Mukolytica oral verabreicht, wobei das feste Arzneimittel in Form von Pulvern, Granulaten oder Brausetabletten vor dem Gebrauch in Wasser gelöst wird.

Als aliphatische Thioverbindungen weist der Wirkstoff einen unangenehmen Geruch und Geschmack und überdies eine hohe Azidität (pKa N-Acetylcystein = 3,24) auf. Um die Akzeptanz bei Patienten zu gewährleisten, werden, Geruch und Geschmack deshalb üblicherweise mit einer grossen Menge an Hilfsstoffen, wie z.B. Saccharose und verschiedensten Aromastoffen, überdeckt.

Da es aber eine Forderung der modernen Arzneiformenlehre ist, kalorische, diabetogene und kariogene Belastungen, die vom Zucker ausgehen, zu reduzieren oder gar zu vermeiden, sind neuerdings Versuche unternommen worden, die Saccharose durch Zuckeralkohole zu ersetzen.

In der europäischen Patentanmeldung mit Veröffentlichungsnummer EP 0 339 508 wird eine N-Acetylcystein enthaltende, im Mund sehr rasch zerfallende und deshalb auch nur für diesen Bereich wirksame Tablette beschrieben, die als Puffer alkalische Hydrogencarbonate und als Geschmacksverbesserer Gemische aus Sorbit/Fructose, Sorbit/Xylit oder Xylit/ Fructose im Gew.-Verhältnis von 0,8:1,2 bis 1,2:0,8 und Aromastoffe enthalten, wobei das Gew.-Verhältnis Kohlenhydrate: N-Acetylcystein 20:1 bis 50:1 beträgt.

In der europäischen Patentanmeldung mit Veröffentlichungsnummer EP 0 340 662 wird ein festes N-Acetylcystein enthaltendes Arzneimittel in Granulatform beschrieben, das als Geschmacksverbesser Xylit, bihydratisiertes Saccharin-Natrium, beta-Carotin und Aromastoffe enthält. Die Menge an Xylit beträgt dabei pro Einheit für 100 mg N-Acetylcystein 1271 mg und für 600 mg N-Acetylcystein 2166 mg.

Weitere Rezepte für Tabletten sind in FR-A-2 452 929 und Manufacturing Chemist 58(6), 55-59 (1987) genannt.

Aus dem Stand der Technik wird ersichtlich, dass es einer geschickten Aromatisierung bedarf, um ein geschmacklich akzeptables Arzneimittel zu erhalten. Dies gilt umso mehr, wenn auf grosse Saccharosemengen bei der Arzneimittelformulierung verzichtet werden soll.

Da gemäss dem Stand der Technik der Anteil an Hilfsstoffen bei N-acetylcysteinhaltigen Arzneimittelformen so gross ist, dass Arzneimittel von 2 bis 3 Gramm Gewicht entstehen, muss das Mittel vor der Einnahme meist in Wasser gelöst oder, wie oben beschrieben, im Mund aufgelöst oder zerkaut werden. Zum Schlucken hingegen eignen sich wegen des dafür notwendigen kleinen Volumens nur Mittel mit geringem Hilfsstoffanteil, was wiederum das Lutschen oder das Trinken des aufgelösten Arzneimittels geruchlich und geschmacklich verunmöglichen würde. Diese je nach Art und Menge der verwendeten Hilfsstoffe eingeschränkte Art der Verabreichung wirkt sich für den Verbraucher nachteilig aus.

Zudem sind in verstärktem Ausmass N-Acetylcystein (Smp. 109-110 °C) ohne vorgängige Granulierstufe kaum zu Tabletten verpressbar, was das Herstellungsverfahren verkompliziert und verlängert.

Aufgabe der vorliegenden Erfindung ist es nun, eine N-acetylcysteinhaltige Arzneimittelzubereitung zur Verfügung zu stellen, die sich aufgrund ihres durch den geringen Anteil an Hilfsstoffen bedingten geringen Volumens zum Schlucken, durch die Art und die Anteile der verwendeten Hilfsstoffe auch zum schnellen Auflösen im Wasser und zum Lutschen eignet und geruchlich und geschmacklich gleichwohl akzeptabel ist und die direkt zu Tabletten verpressbar ist.

Die Aufgabe wird gemäß Anspruch 1 gelöst.

Durch die Wahl der geeigneten Hilfsstoffe können dadurch die Bestandteile des Gemisches auf übliche Art direkt zu Tabletten verpresst werden, ohne vorgängig einer Granulierungsstufe unterworfen worden zu sein. Dies vereinfacht in wesentlichem Ausmass die Herstellung der erfindungsgemässen Tablette.

Eine Arzneimittelzubereitung der vorbeschriebenen Art in Tablettenform mit weniger als 50 Gew.-% Hilfsstoffe, bezogen auf das Gesamtgewicht der Zubereitung, stellt eine kostengünstige Arzneimittelzubereitung dar.

Trotz der geringen Menge an verwendeten Hilfsstoffen lässt die Kombination der Hilfsstoffe überraschenderweise eine geruchlich und geschmacklich akzeptable und im Verhältnis zum Stand der Technik hochkonzentrierte N-acetylcysteinhaltige Arzneimittelzubereitung zu.

N-Acetylcystein kann jeweils allein oder in Kombination von 1:99 bis 99:1 Teilen miteinander verwendet werden.

Als Cellulose eignet sich besonders mikrokristalline Cellulose, die wegen ihrer überraschend guten Verpresseigenschaften für N-Acetylcystein für eine direkte Verpressung der Mischung zu Tabletten verwendet werden kann. Bezogen auf N-Acetylcystein beträgt ihr Anteil vorzugsweise 0,1 bis 25 Gew.-%, vorzugsweise 15 bis 25 Gew.-%.

Als Cellulosederivat eignet sich vorzüglich quervernetzte Natriumcarboxymethylcellulose (NaCMC), die für die rasche Auflösung der Tablette förderlich ist, und deren Anteil 0,1 bis 4 Gew.-%, vorzugsweise 4 bis 12 Gew.-% bezogen auf N-Acetylcystein beträgt.

Als Zuckeralkohol eignet sich besonders Mannit, das sowohl zur Geschmacksverbesserung beiträgt als auch überraschend ein Nachhärten der Tablette während der Lagerung verhindert. Dadurch wird eine Verzögerung der Auflösungsgeschwindigkeit der Tablette im Laufe der Lagerung vermieden. Das Verhältnis Mannit zu N-Acetylcystein beträgt vorzugsweise 2 bis 35 Gew.-%, vorzugsweise 25 bis 35 Gew.-%.

Als Süssstoffe eignen sich besonders Mischungen von Aspartam und Acesulfam-Kalium im Verhältnis von 5:1 bis 1:5, vorzugsweise 3:1 bis 1:1. Ueberraschenderweise werden, bezogen auf N-Acetylcystein, nur 0,1 bis 12 Gew.-% Süssstoff, vorzugsweise 4 bis 12 Gew.-%, benötigt, um den Geschmack der Arzneimittelzubereitung entscheidend zu verbessern.

Für den Geschmack hat sich eine Aromatisierung mit Zitronen-, Grapefruit- und/oder Pinacoladaaromastoffen als besonders geeignet erwiesen. Im Verhältnis zu N-Acetylcystein wird vorzugsweise 0,1 bis 8 Gew. -%, besonders bevorzugt 2 bis 8 Gew.-%, mindestens eines Fruchtaromastoffes verwendet. Damit kann der dem N-Acetylcystein anhaftende übelriechende und -schmeckende Schwefelwasserstoffduft und -geschmack sowohl bei der in Wasser gelösten als auch bei der gelutschten Tablette überdeckt werden.

Als weitere Tablettierhilfsmittel eignen sich z.B. Magnesiumstearat und/oder kolloidale Kieselsäure, die den Tabletten zwecks besserer Verpressbarkeit beigemischt werden können. Es werden vorzugsweise Mengen von jeweils 0,1 bis 8 Gew.-%, vorzugsweise 1 bis 4 Gew.-%, bezogen auf N-Acetylcystein gewählt, damit eine kolloidale Lösung der Tabletten noch gewährleistet werden kann.

Die erfindungsgemässen Tabletten lösen sich in höchstens 2 Minuten, meist jedoch schon in weniger als 1 Minute klar bis kolloidal in Wasser oder in wässriger Lösung auf; sie können jedoch auch ganz geschluckt oder im Mund gelutscht werden, wobei sie im letzteren Fall in wenigen Sekunden bis höchstens einer Minute zerfallen, so dass N-Acetylcystein seine Wirkung in kürzester Zeit entfalten kann. Dabei trägt der erfrischende, säuerliche Geschmack mit Fruchtcharakter, vor allem auch bei gereizter Schleimhaut im Mund-Rachenraum, zum Wohlbefinden bei.

Die im folgenden beschriebenen Arzneimittelzubereitungen werden in an sich bekannter Weise hergestellt.

### Beispiel 1

Folgende pulverförmige Komponenten werden miteinander homogen gemischt:

| | mg pro Tablette |
|---|---|
| N-Acetylcystein | 100 |
| Mikrokristalline Cellulose | 20 |
| Mannit | 30 |
| NaCMC | 7,5 |
| Aspartam | 4,5 |
| Acesulfam-Kalium | 2,0 |
| Kolloidale Kieselsäure | 2,0 |
| Magnesiumstearat | 1,5 |
| Zitronensaftaroma | 4,0 |
| Total | 171,5 |

Die Mischung wird direkt zu Tabletten mit einem grössten Durchmesser von 7 mm verpresst.

Die Auflösungszeit der Tablette in Wasser von 20 °C liegt unter einer Minute.

### Beispiel 2

Folgende pulverförmige Komponenten werden miteinander homogen gemischt:

| | mg pro Tablette |
|---|---|
| N-Acetylcystein | 200 |
| Mikrokristalline Cellulose | 40 |
| Mannit | 60 |
| NaCMC | 15 |
| Aspartam | 9 |
| Acesulfam-Kalium | 4 |
| Kolloidale Kieselsäure | 4 |
| Magnesiumstearat | 3 |
| Grapefruitaroma | 8 |
| Total | 343 |

Die Mischung wird direkt zu Tabletten mit einem grössten Durchmesser von 10 mm verpresst.

Die Auflösungszeit der Tablette in Wasser von 20 °C liegt unter einer Minute.

### Beispiel 5

Bei einem Vergleichsversuch mit einer Zubereitung aus einem herkömmlichen N-Acetylcystein-Granulat an 10 Probanden hat sich überraschenderweise gezeigt, dass die erfindungsgemässe Arzneimittelzubereitung zu einer besseren Bioverfügbarkeit des Wirkstoffs (höhere AUC) führt. Dabei wurde ein Verfahren zur quantitativen Bestimmung von N-Acetylcystein im Plasma verwendet, das den Wirkstoff mit Tributylphosphin reduktiv spaltet und mit einer post-HPLC-Säule mittels o-Phthalaldehyd derivatisiert.

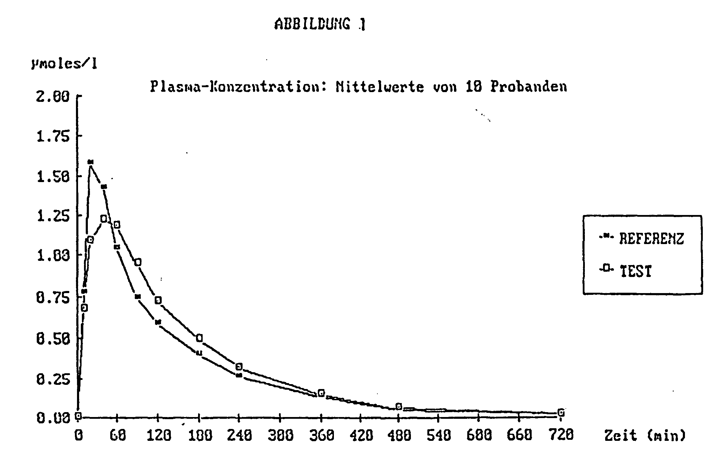

Durch dieses Verfahren wurden zum ersten Mal endogene Durchschnittskonzentrationen von 0,08 µM gemessen. Der Wirkstoff wurde im Plasma noch nach 12 Stunden nach oraler Applikation einer einmaligen Dosis von 200 mg gefunden. Die Cₘₐₓ-Werte waren bis zu 20 mal höher als die endogenen Plasmawerte der Testpersonen (vgl. Abbildung 1 und Tabelle 1). Der Vergleichsversuch ist in Biopharmaceutics & Drug Disposition, Vol. 12, 343-353 (1991) dokumentiert.

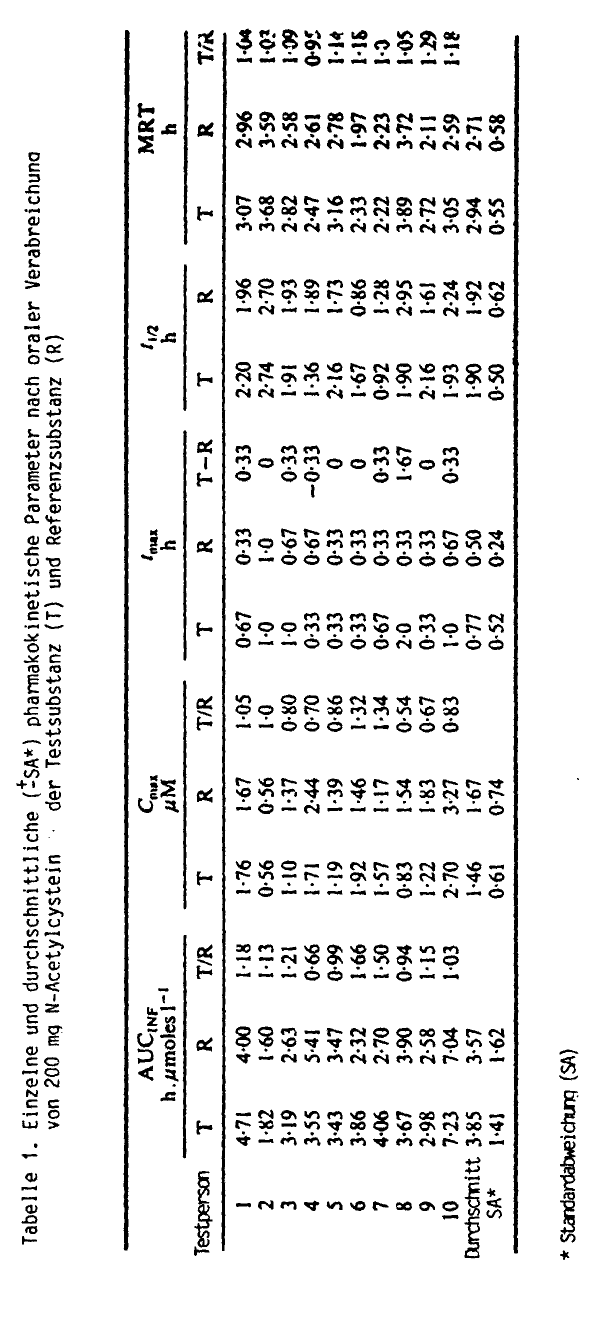

## Patentansprüche (Patentansprüche für folgende(n) Vertragsstaat(en): AT, BE, CH, LI, DE, DK, FR, GB, IT, LU, NL, SE)

1. Oral verabreichbare, schnell-lösliche, N-Acetylcystein-haltige feste Arzneimittelzubereitung, deren gewichtsmässiger Anteil an Wirkstoff mindestens 50 Gew.-% des Gesamtgewichts der Arzneimittelzubereitung ausmacht und die einen Anteil an Hilfsstoffen bezogen auf N-Acetylcystein, von 15-25 Gew.-% Cellulose und/oder 4-12 Gew.-% Cellulosederivate, 25-35 Gew.-% Zuckeralkohol, 4-12 Gew.-% Süssstoff, 2-8 Gew.-% Aromastoffe und gegebenenfalls 2-8 Gew.-% Tablettierhilfsmittel aufweist.

2. Arzneimittelzubereitung nach Patentanspruch 1, die 50 bis 400 mg N-Acetylcystein enthält und einen Durchmesser von 5 bis 12 mm aufweist.

3. Arzneimittelzubereitung nach Patentanspruch 1 oder 2, dadurch gekennzeichnet, dass als Cellulose mikrokristalline Cellulose verwendet wird.

4. Arzneimittelzubereitung nach einem der Patentansprüche 1-3, dadurch gekennzeichnet, dass als Cellulosederivat Natriumcarboxymethylcellulose verwendet wird.

5. Arzneimittelzubereitung nach einem der Patentansprüche 1-4, dadurch gekennzeichnet, dass als Zuckeralkohol Mannit verwendet wird.

6. Arzneimittelzubereitung nach einem der Patentansprüche 1-5, dadurch gekennzeichnet, dass als Süssstoff Mischungen von Aspartam und Acesulfam-Kalium im Verhältnis 5:1 bis 1:5, vorzugsweise 3:1 bis 1:1, verwendet werden.

7. Arzneimittelzubereitung nach einem der Patentansprüche 1-6, dadurch gekennzeichnet, dass als Aromastoffe mindestens ein Fruchtaroma, wie Zitronensaft-, Grapefruitsaft- oder Pinacoladaaroma, verwendet wird.

8. Arzneimittelzubereitung nach einem der Patentansprüchel-7,dadurch gekennzeichnet, dass als Tablettierhilfsmittel eine kolloidal lösliche Menge mindestens eines Gleitmittels, vorzugsweise Magnesiumstearat und/oder kolloidale Kieselsäure, verwendet wird.

9. Arzneimittelzubereitung nach einem der Patentansprüche 1-8 enthaltend 100 mg N-Acetylcystein, 20 mg mikrokristalline Cellulose, 30 mg Mannit, 7,5 mg Natrium-carboxymethylcellulose, 4,5 mg Aspartam, 2,0 mg Acesulfam-Kalium, 2,0 mg kolloidale Kieselsäure, 1,5 mg Magnesiumstearat, 4,0 mg Zitronensaft Aroma.

10. Arzneimittelzubereitung nach einem der Patentansprüche 1-9, enthaltend 200 mg N-Acetylcystein, 40 mg mikrokristalline Cellulose, 60 mg Mannit, 15 mg Natriumcarboxymethylcellulose, 9 mg Aspartam, 4 mg Acesulfam-Kalium, 4 mg kolloidale Kieselsäure, 3 mg Magnesiumstearat, 8 mg Grapefruit Aroma.

11. Verfahren zur Herstellung einer Arzneimittelzubereitung nach einem der Patentansprüche 1-10, dadurch gekennzeichnet, dass N-Acetylcystein mit den Hilfsstoffen direkt zu Tabletten verpresst werden.

12. Schluck-, lutsch- und in wässriger Lösung auflösbare Arzneimittelzubereitung nach einem der Patentansprüche 1-10.

13. Innerhalb einer Minute beim Lutschen zerfallende oder in Wasser oder wässriger Lösung sich auflösende Arzneimittelzubereitung nach einem der Patentansprüche 1-10 und 12.

## Patentansprüche (Patentansprüche für folgende(n) Vertragsstaat(en): ES, GR)

1. Verfahren zur Herstellung einer oral verabreichbaren, schnell-löslichen, N-Acetylcystein-haltigen festen Arzneimittelzubereitung, dadurch gekennzeichnet, dass man N-Acetylcystein dessen gewichtsmässiger Anteil mindestens 50 Gew.-% des Gesamtgewichts der Arzneimittelzubereitung ausmacht, und die Hilfsstoffe, welche bezogen auf N-Acetylcystein 15-25 Gew-% Cellulose und/oder 4-12 Gew.-% Cellulosederivat, 25-35 Gew.-% Zuckeralkohol, 4-12 Gew.-% Süssstoff, 2-8 Gew.-% Aromastoff und gegebenenfalls 2-8 Gew-% Tablettierhilfsmittel enthalten, direkt zu Tabletten verpresst.

2. Verfahren nach einem der Patentanspruch 1, dadurch gekennzeichnet, dass die Arzneimittelzubereitung 50 bis 400 mg N-Acetylcystein enthält und einen Durchmesser von 5 bis 12 mm aufweist.

3. Verfahren nach einem der Patentanspruch 1 oder 2, dadurch gekennzeichnet, dass als Cellulose mikrokristalline Cellulose verwendet wird.

4. Verfahren nach einem der Patentansprüche 1-3, dadurch gekennzeichnet, dass als Cellulosederivat Natriumcarboxymethylcellulose verwendet wird.

5. Verfahren nach einem der Patentansprüche 1-4, dadurch gekennzeichnet, dass als Zuckeralkohol Mannit verwendet wird.

6. Verfahren nach einem der Patentansprüche 1-5, dadurch gekennzeichnet, dass als Süssstoff Mischungen von Aspartam und Acesulfam-Kalium im Verhältnis 5:1 bis 1:5, vorzugsweise 3:1 bis 1:1, verwendet werden.

7. Verfahren nach einem der Patentansprüche 1-6, dadurch gekennzeichnet, dass als Aromastoffe mindestens ein Fruchtaroma, wie Zitronensaft-, Grapefruitsaft- oder Pinacoladaaroma, verwendet wird.

8. Verfahren nach einem der Patentansprüche 1-7, dadurch gekennzeichnet, dass als Tablettierhilfsmittel eine kolloidal lösliche Menge mindestens eines Gleitmittels, vorzugsweise Magnesiumstearat und/oder kolloidale Kieselsäure, verwendet wird.

9. Verfahren nach einem der Patentansprüche 1-8, dadurch gekennzeichnet, dass die Arzneimittelzubereitung 100 mg N-Acetylcystein, 20 mg mikrokristalline Cellulose, 30 mg Mannit, 7,5 mg Natriumcarboxymethylcellulose, 4,5 mg Aspartam, 2,0 mg Acesulfam-Kalium, 2,0 mg kolloidale Kieselsäure, 1,5 mg Magnesiumstearat, 4,0 mg Zitronensaft Aroma enthält.

10. Verfahren nach einem der Patentansprüche 1-8, dadurch gekennzeichnet, dass die Arzneimittelzubereitung 200 mg N-Acetylcystein 40 mg mikrokristalline Cellulose, 60 mg Mannit, 15 mg Natriumcarboxymethylcellulose, 9 mg Aspartam, 4 mg Acesulfam-Kalium, 4 mg kolloidale Kieselsäure, 3 mg Magnesiumstearat, 8 mg Grapefruit Aroma enthält.

11. Verfahren zur Herstellung einer schluck-, lutsch- und in wässriger Lösung auflösbare Arzneimittelzubereitung nach einem der Patentansprüche 1-10.

12. Verfahren zur Herstellung einer innerhalb einer Minute beim Lutschen zerfallenden oder in Wasser oder wässriger Lösung sich auflösenden Arzneimittelzubereitung nach einem der Patentansprüche 1-10.

## Claims (Claims for the following Contracting State(s): AT, BE, CH, LI, DE, DK, FR, GB, IT, LU, NL, SE)

1. An orally administered, fast-dissolving, solid drug preparation containing N-acetylcysteine, whose proportion by weight of active ingredient is at least 50% of the total weight of the drug preparation and whose remaining amount contains as inactive ingredients in relation to N-acetylcysteine by weight 15-25% cellulose and/or 4-12% cellulose derivatives, 25-35% sugar alcohol, 4-12% sweetener, 2-8% flavoring and, if need be, 2-8% tableting adjuvant.

2. A drug preparation according to Patent Claim 1, which contains 50 to 400 mg N-acetylcysteine and has a diameter of 5 to 12 mm.

3. A drug preparation according to one of Patent Claims 1 or 2, characterized by the fact that microcrystalline cellulose is used as the cellulose.

4. A drug preparation according to one of Patent Claims 1-3, characterized by the fact that sodium carboxymethyl cellulose is used as a cellulose derivative.

5. A drug preparation according to one of Patent Claims 1 to 4, characterized by the fact that mannitol is used as a sugar alcohol.

6. A drug preparation according to one of Patent Claims 1 to 5, characterized by the fact that mixtures of aspartame and acesulfam potassium are used as sweeteners in a ratio of 5:1 to 1:5, preferably 3:1 to 1:1.

7. A drug preparation according to one of Patent Claims 1 to 6, characterized by the fact that at least one fruit flavor, like lemonade, grapefruit juice or pina colada flavor is used as a flavoring.

8. A drug preparation according to one of Patent Claims 1 to 7, characterized by the fact that a colloidal soluble amount of at least one lubricant, preferably magnesium stearate and/or colloidal silicic acid is used as a tableting adjuvant.

9. A drug preparation according to one of Patent Claims 1 to 8, containing 100 mg of N-acetylcysteine, 20 mg of microcrystalline cellulose, 30 mg of mannitol, 7.5 mg of sodium carboxymethyl cellulose, 4.5 mg of aspartame, 2.0 mg of acesulfam potassium 2.0 mg of colloidal silicic acid, 1.5 mg of magnesium stearate and 4.0 mg of lemonade flavoring.

10. A drug preparation according to one of Patent Claims 1 to 9, containing 200 mg of N-acetylcysteine 40 mg of microcrystalline cellulose, 60 mg of mannitol, 15 mg of sodium carboxymethyl cellulose, 9 mg of aspartame, 4 mg of acesulfam potassium, 4 mg of colloidal silicic acid, 3 mg of magnesium stearate and 8 mg of grapefruit flavoring.

11. A processing for producing a drug preparation according to one of Patent Claims 1 to 10, characterized by the fact that N-acetylcysteine is compressed directly into tablets with the inactive ingredients.

12. A drug preparation that can be swallowed, sucked and dissolved in aqueous solution according to one of Patent Claims 1 to 10.

13. A drug preparation according to one of Patent Claims 1 to 10 and 12 that disintegrates when sucked or dissolves in water or an aqueous solution within one minute.

## Claims (Claims for the following Contracting State(s): ES, GR)

1. A processing for producing an orally administered, fast-dissolving, solid drug preparation containing N-acetylcysteine, characterized by the fact that N-acetylcysteine, whose proportion by weight of active ingredient is at least 50% of the total weight of the drug preparation and the inactive ingredients which contain in relation to N-acetylcysteine 15-25% cellulose and/or 4-12% cellulose derivative, 25-35% sugar alcohol, 4-12% sweetener, 2-8% flavoring and, if need be, 2-8% tableting adjuvant are compressed directly into tablets.

2. A processing according to Patent Claim 1, characterized by the fact that the drug preparation contains 50 to 400 mg N-acetylcysteine and has a diameter of 5 to 12 mm.

3. A processing according to one of Patent Claims 1 or 2, characterized by the fact that microcrystalline cellulose is used as the cellulose.

4. A processing according to one of Patent Claims 1 to 3, characterized by the fact that sodium carboxymethyl cellulose is used as a cellulose derivative.

5. A processing according to one of Patent Claims 1 to 4, characterized by the fact that mannitol is used as a sugar alcohol.

6. A processing according to one of Patent Claims 1 to 5, characterized by the fact that mixtures of aspartame and acesulfam potassium are used as sweeteners in a ratio of 5:1 to 1:5, preferably 3:1 to 1:1.

7. A processing according to one of Patent Claims 1 to 6, characterized by the fact that at least one fruit flavor, like lemonade, grapefruit juice or pina colada flavor is used as a flavoring.

8. A processing according to one of Patent Claims 1 to 7, characterized by the fact that a colloidal soluble amount of at least one lubricant, preferably magnesium stearate and/or colloidal silicic acid is used as a tableting adjuvant.

9. A processing according to one of Patent Claims 1 to 8, characterized by the fact that the drug preparation contains 100 mg of N-acetylcysteine, 20 mg of microcrystalline cellulose, 30 mg of mannitol, 7,5 mg of sodium carboxymethyl cellulose, 4,5 mg of aspartame, 2,0 mg of acesulfam potassium, 2,0 mg of colloidal silicic acid, 1,5 mg of magnesium stearate and 4,0 mg of lemonade flavoring.

10. A processing according to one of Patent Claims 1 to 8, characterized by the fact that the drug preparation contains 200 mg of N-acetylcysteine, 40 mg of microcrystalline cellulose, 60 mg of mannitol, 15 mg of sodium carboxymethyl cellulose, 9 mg of aspartame, 4 mg of acesulfam potassium, 4 mg of colloidal silicic acid, 3 mg of magnesium stearate and 8 mg of grapefruit flavoring.

11. A processing for producing a drug preparation according to one of Patent Claims 1 to 10 that can be swallowed, sucked and dissolved in aqueous solution.

12. A processing for producing a drug preparation according to one of Patent Claims 1 to 10 that disintegrates when sucked or dissolves in water or an aqueous solution within one minute.

## Revendications (Revendications pour l'(les) Etat(s) contractant(s) suivant(s): AT, BE, CH, LI, DE, DK, FR, GB, IT, LU, NL, SE)

1. Préparation pharmaceutique solide, contenant de la N-acétylcystéine, pour administration orale, à dissolution rapide, dont la quantité pondérale de matière active atteint au moins 50% en poids du poids total de la préparation pharmaceutique et dont la quantité restante contient, comme excipient, sur la base de N-acétylcystéine 15-25% en poids de cellulose et/ou 4-12% en poids d'un dérivé de la cellulose, au 25-35% en poids d'alcool de sucre, 4-12% en poids d'édulcorant, 2-8% en poids d'un agent d'arôme et facultativement, 2-8% en poids d'additif de comprimé.

2. Préparation pharmaceutique suivant la revendication 1 qui contient 50 à 400 mg de N-acétylcystéine et présente un diamètre de 5 à 12 mm.

3. Préparation pharmaceutique suivant l'une quelconque des revendications 1 et 2, caractérisée en ce que l'on utilise de la cellulose microcristalline comme cellulose.

4. Préparation pharmaceutique suivant l'une quelconque des revendications 1 à 3, caractérisée en ce que l'on utilise de la carboxyméthylcellulose sodique comme dérivé de la cellulose.

5. Préparation pharmaceutique suivant l'une quelconque des revendications 1 à 4, caractérisée en ce que l'on utilise du mannitol comme alcool de sucre.

6. Préparation pharmaceutique suivant l'une quelconque des revendications 1 à 5, caractérisée en ce que l'on utilise des mélanges d'aspartame et d'acésulfame de potassium en proportion 5:1 à 1:5, de préférence, 3:1 à 1:1, comme édulcorant.

7. Préparation pharmaceutique suivant l'une quelconque des revendications 1 à 6, caractérisée en ce que l'on utilise au moins un arôme de fruit, à savoir, arôme de citron, de pamplemousse ou de pina colada, comme arôme.

8. Préparation pharmaceutique suivant l'une quelconque des revendications 1 à 7, caractérisée en ce que l'on utilise une quantité colloïdale soluble d'au moins un agent de coulance, de préférence, du stéarate de magnésium et/ou e l'acide silicique colloïdale, comme additif de comprimé.

9. Préparation pharmaceutique suivant l'une quelconque des revendications 1 à 8, contenant 100 mg de N-acétylcystéine, 20 mg de cellulose microcristalline, 30 mg de mannitol, 7,5 mg de carboxyméthylcellulose sodique, 4,5 mg d'aspartame, 2,0 mg d'acésulfame de potassium, 2,0 mg d'acide silicique colloïdale, 1,5 mg de stéarate de magnésium, 4,0 mg d'arôme de citron.

10. Préparation pharmaceutique suivant l'une quelconque des revendications 1 à 9, contenant 200 mg de N-acétylcystéine, 40 mg de cellulose microcristalline, 60 mg de mannitol, 15 mg de carboxyméthylcellulose sodique. 9 mg d'aspartame, 4 mg d'acésulfame de potassium, 4 mg d'acide siliciquecolloïdale, 3 mg de stéarate de magnésium, 8 mg d'arôme de pamplemousse.

11. Procédé de préparation d'une préparation pharmaceutique suivant l'une quelconque des revendications 1 à 10, caractérisé en ce que la N-acétylcystéine ainsi que les excipients sont directement compressés en comprimés.

12. Préparation pharmaceutique à avaler ou à sucer et pouvant être dissoute en solution aqueuse suivant l'une quelconque des revendications 1 à 10.

13. Préparation pharmaceutique suivant l'une quelconque des revendications 1 à 10 et 12, pouvant être désintégrée lors du sucement ou dissoute dans l'eau ou dans une solution aqueuse en l'espace d'une minute.

## Revendications (Revendications pour l'(les) Etat(s) contractant(s) suivant(s): ES, GR)

1. Procédé de préparation d'une préparation pharmaceutique solide, contenant de la N-acétylcystéine, pour administration orale, à dissolution rapide, caractérisé en ce que l'on compresse directement en comprimés, la N-acétylcystéine, dont la quantité pondérale atteint au moins 50% en poids du poids total de la préparation pharmaceutique et l'excipient qui contient sur la base de N-acétylcysteine 15-25% en poids de cellulose et/ou 4-12% en poids d'un dérivé de la cellulose, 25-35% en poids d'alcool de sucre, 4-12% en poids d'édulcorant, 2-8% en poids d'un agent d'arôme et facultativement, 2-8% en poids d'additif de comprimé.

2. Procédé suivant la revendication 1, caractérisé en ce que la préparation pharmaceutique contient 50 à 400 mg de N-acétylcystéine et présente un diamètre de 5 à 12 mm.

3. Procédé suivant l'une quelconque des revendications 1 et 2, caractérisé en ce que l'on utilise de la cellulose microcristalline comme cellulose.

4. Procédé suivant l'une quelconque des revendications 1 à 3, caractérisé en ce que l'on utilise de la carboxyméthylcellulose sodique comme dérivé de la cellulose.

5. Procédé suivant l'une quelconque des revendications 1 à 4, caractérisé en ce que l'on utilise du mannitol comme alcool de sucre.

6. Procédé suivant l'une quelconque des revendications 1 à 5, caractérisé en ce que l'on utilise des mélanges d'aspartame et d'acésulfame de potassium en proportion 5:1 à 1:5, de préférence, 3:1 à 1:1, comme édulcorant.

7. Procédé suivant l'une quelconque des revendications 1 à 6, caractérisé en ce que l'on utilise au moins un arôme de fruit, à savoir, arôme de citron, de pamplemousse ou de pina colada, comme agent d'arôme.

8. Procédé suivant l'une quelconque des revendications 1 à 7, caractérisé en ce que l'on utilise une quantité colloïdale soluble d'au moins un agent de coulance, de préférence, du stéarate de maqnésium et/ou de l'acide silicique colloïdale, comme additif de comprimé.

9. Procédé suivant l'une quelconque des revendications 1 à 8, caractérisé en ce que la préparation pharmaceutique contient 100 mg de N-acétylcystéine, 20 mg de cellulose microcristalline, 30 mg de mannitol, 7,5 mg de carboxyméthylcellulose sodique, 4,5 mg d'aspartame, 2,0 mg d'acésulfame de potassium, 2,0 mg d'acide silicique colloïdale 1,5 mg de stéarate de magnésium, 4,0 mg d'arôme de citron.

10. Procédé suivant l'une quelconque des revendications 1 à 8, caractérisé en ce que la préparation pharmaceutique contient 200 mg de N-acétylcystéine, 40 mg de cellulose microcristalline, 60 mg de mannitol, 15 mg de carboxyméthylcellulose sodique, 9 mg d'aspartame, 4 mg d'acésulfame de potassium, 4 mg d'acide silicique colloidale, 3 mg de stéarate de magnésium, 8 mg d'arôme de pamplemousse.

11. Procédé de préparation d'une préparation pharmaceutique à avaler ou à sucer et pouvant être dissoute en solution aqueuse suivant l'une quelconque des revendications 1 à 10.

12. Procédé de préparation d'une préparation pharmaceutique suivant l'une quelconque des revendications 1 à 10, pouvant être désintégrée lors du sucement ou dissoute dans l'eau ou dans une solution aqueuse en l'espace d'une minute.
